# EUROPEAN PATENT APPLICATION

(11) **EP 4 516 330 A1**
(43) Date of publication of application: **05.03.2025**
(21) Application number: 23796510.8
(22) Date of filing: 27.04.2023
(51) Int. Cl.: A61L 27/16, A61L 27/20, A61L 27/22, A61L 27/36, A61L 27/38, A61L 27/52

(54) **IMMUNOISOLATION DEVICE**

(30) Priority: 28.04.2022 JP 2022075135
(71) Applicant: Kuraray Co., Ltd., Kurashiki-shi, Okayama 710-0801 (JP); Tokyo Women's Medical University, Tokyo 162-8666 (JP)
(72) Inventor: KOBAYASHI, Goro, Tsukuba-shi, Ibaraki 305-0841 (JP); AYANO, Satoru, Tsukuba-shi, Ibaraki 305-0841 (JP); FUJITA, Akio, Tsukuba-shi, Ibaraki 305-0841 (JP); HOMMA, Jun, Tokyo 162-8666 (JP); SEKINE, Hidekazu, Tokyo 162-8666 (JP); SHIMIZU, Tatsuya, Tokyo 162-8666 (JP)
(74) Representative: Müller-Boré & Partner Patentanwälte PartG mbB
(86) International application number: PCT/JP2023/016742
(87) International publication number: WO 2023/210775

(57) **Abstract**

The problem to be solved by the present invention is to provide an immunoisolation device that achieves both a reduction in diffusion distance, which is effective for increasing the permeability of substances such as physiologically active substances and nutrients, and improvement in durability to withstand long-term transplantation. Another object of the present invention is to provide an immune control technique using a cell sheet. The present invention provides an immunoisolation device comprising a sheet-like cell aggregate containing cells and an extracellular matrix, and an immunoisolation layer that covers the cell aggregate.

## Description

### Technical Field

### Cross-Reference to Related Applications

This application claims priority based on Japanese Patent Application No. 2022-075135 filed on April 28, 2022 (the entire disclosure of which is incorporated herein by reference). The present invention relates to an immunoisolation device.

### Background Art

Immunoisolation devices have been developed as a means for performing cell transplantation therapy without the need to administer an immunosuppressant. In particular, for example, in transplantation of somatic cells derived from iPS cells, for which the risk of canceration is a concern, or at the time of a decrease in the function of transplanted cells, macroencapsulation immunoisolation devices are considered an effective method in that the transplantation site can be identified and that the devices can be replaced. Important functions required of macroencapsulation immunoisolation devices are the following: cells or cell clusters can be uniformly dispersed and fixed without incurring association of cells or cell clusters; the devices allow for easy permeation of oxygen and/or nutritional components to transplanted cells; the devices allow for easy release of a desired physiologically active substance (cytokines, hormones, growth factors, etc.) from cells, which is required for a therapeutic effect, according to cell response; permeation of immunoresponsive cells and immune response factors is prevented; and the transplanted devices are excellent in biocompatibility, and are less likely to adhere to surrounding tissue and less likely to induce inflammatory responses, such as granulation.

### Citation List

### Patent Literature

PTL 1: JP2012-508584A
PTL 2: Japanese Patent No. 5717253

### Summary of Invention

### Technical Problem

Many immunoisolation devices using porous membranes have been studied (Patent Literature (PTL) 1) and have been considered to have one problem, that is a decrease in permeability due to protein adsorption onto porous membrane materials or fibrosis, or a decrease in permeability due to adhesion to surrounding tissue. Internal necrosis of cell clusters etc. is considered to be induced when the diameter exceeds 500 um. In order to easily maintain engraftment of embedded cells to a recipient and release physiologically active substances, it is necessary to appropriately control the overall thickness of such an immunoisolation device, and it is desirable that the immunoisolation layer constituting the device is as thin as possible. On the other hand, for transplantation into the body, it is necessary to improve the membrane strength and durability to prevent the device from breaking or being twisted. However, it is not easy to achieve both a reduction in membrane thickness and improvement in durability.

Further, a temperature-responsive culture dish can be used to culture cells for transplantation, which makes it possible to produce cells in the form of a single sheet containing an extracellular matrix, an adhesion factor, etc. For example, a myocardial sheet, which is used by applying a skeletal muscle cell sheet as a "cell sheet" to an affected site, has been put into practical use as a regenerative medicine product. Further, Patent Literature (PTL) 2 discloses a method for producing a cell sheet of pancreatic islet cells and a method for use thereof. An advantage of cell sheets is that a cell sheet comprises an extracellular matrix, a cell adhesion factor, etc., and achieves excellent cell engraftment in transplantation without excessive progress of cell cluster formation due to cell association.

However, the cell sheet transplantation method is currently only put into practical used in autologous cells. When allogeneic cells are used, the cell sheet is not engrafted into the host, but dies due to an immune rejection reaction. Therefore, the administration of an immunosuppressant is essential. However, since HLA homology between the donor and the host is essential, there are many challenges to overcome before this method is popularized and commercialized as a regenerative medicine technique. The Examples of Patent Literature (PTL) 2 also disclose transplantation into the same species of rats, and PLT 2 further discloses examples of transplantation into immunodeficient mice; however, no immune control techniques were used therein.

An object of the present invention is to provide an immunoisolation device that achieves both a reduction in diffusion distance, which is effective for increasing the permeability of substances such as physiologically active substances and nutrients, and improvement in durability to withstand long-term transplantation. Another object of the present invention is to provide an immune control technique using a cell sheet.

### Solution to Problem

The present invention provides the following immunoisolation devices.
[1] An immunoisolation device comprising a sheet-like cell aggregate and an immunoisolation layer,
   the sheet-like cell aggregate comprising cells and an extracellular matrix, and
   the immunoisolation layer covering the cell aggregate.
[2] The immunoisolation device according to [1], wherein the cell aggregate is a cell sheet comprising the cells and the extracellular matrix.
[3] The immunoisolation device according to [1] or [2], wherein the cell aggregate has a thickness of 300 µm or less.
[4] The immunoisolation device according to any one of [1] to [3], wherein the immunoisolation layer comprises a porous membrane or a fiber structure,
   the porous membrane or the fiber structure comprises at least one member selected from the group consisting of ethylene-vinyl alcohol copolymers and cellulose acetate.
[5] The immunoisolation device according to [4], wherein the immunoisolation layer is an immunoisolation multilayer comprising the porous membrane or the fiber structure, and a hydrogel.
[6] The immunoisolation device according to [5], wherein the outermost layer of the immunoisolation multilayer is the porous membrane or the fiber structure, and the innermost layer is the hydrogel.
[7] The immunoisolation device according to [5], wherein
   the outermost layer of the immunoisolation multilayer is the hydrogel and
   the innermost layer of the immunoisolation multilayer is the porous membrane or the fiber structure.
[8] The immunoisolation device according to any one of [1] to [7], wherein
   the immunoisolation layer comprises multiple layers, and
   the innermost layer of the immunoisolation layer is a hydrogel or a porous membrane having a cell-adhesive protein and/or a cell-adhesive peptide immobilized.
[9] The immunoisolation device according to any one of [5] to [8], wherein the hydrogel comprises at least one member selected from the group consisting of polyvinyl alcohols and polyethylene glycol.

### Advantageous Effects of Invention

The present invention can provide an immunoisolation device that achieves both a reduction in diffusion distance, which is effective in increasing the permeability of substances such as physiologically active substances and nutrients, and improvement in durability to withstand long-term transplantation. Further, the present invention can provide an immune control technique using a cell sheet.

### Brief Description of Drawings

Fig. 1 is a perspective view of a bag-shaped immunoisolation device.
Fig. 2 is a cross-sectional view of a tubular immunoisolation device.
Fig. 3 is a conceptual diagram of a cell sheet.
Fig. 4 is a conceptual diagram of a device having a cell sheet enclosed therein.
Fig. 5 is a cross-sectional view of an immunoisolation layer obtained by multilayer formation using a porous membrane and a hydrogel.
Fig. 6 is a cross-sectional view of an immunoisolation layer obtained by multilayer formation using a porous membrane and a hydrogel.
Fig. 7 is an SEM image of a cross-section of the immunoisolation layer (B) obtained in Production Example 1.
Fig. 8 is a schematic diagram of an instrument for measuring the permeation amounts of glucose, insulin, immune system humoral factors, and the like permeated through the immunoisolation layer.
Fig. 9 is a conceptual diagram of the device obtained in Example 1.
Fig. 10 is a graph of the functionality evaluation performed in Example 1.
Fig. 11 shows results of the histopathological evaluation performed in Example 1

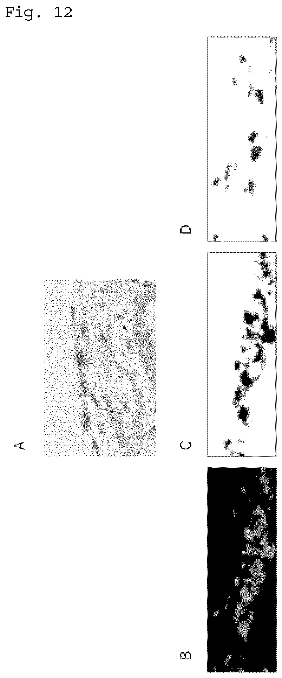

Fig. 12 shows results of the histopathological evaluation performed in Example 1. Fig. 12A is a histological staining image. 12B to 12D are fluorescent staining images. Fig. 12B is the original image. Fig. 12C is an image in which insulin-positive areas (anti-insulin (pancreatic islet β cells) in the original image (Fig. 12B) are shown in black. Fig. 12D is an image in which cell nucleus portions (Hoechst dye-stained portions) in the original picture (Fig. 12B) are shown in black. As shown in Figs. 12B to 12D, cell nuclei were observed in the insulin-positive areas in the fluorescent stained image.

### Description of Embodiments

The singular forms ("a," "an," and "the") used in the present specification are intended to include both the singular and the plural unless otherwise specified herein or clearly contradictory in context.

The immunoisolation device of the present invention comprises a sheet-like cell aggregate and an immunoisolation layer, wherein the sheet-like cell aggregate comprises cells and an extracellular matrix, and the cell aggregate is covered with the immunoisolation layer to thereby suppress the invasion of immune cells and cytokines into the cell aggregate. The immunoisolation layer is preferably an immunoisolation multilayer comprising a porous membrane or fiber structure, and a hydrogel.

### Cell Aggregate

The cell aggregate to be used in the immunoisolation device of the present invention is a sheet-like cell aggregate comprising cells and an extracellular matrix. Examples of cell aggregates include a composite of cells and an extracellular matrix; cells immobilized on a sheet formed using a collagen sheet or collagen sponge as a scaffolding material; and a cell sheet prepared using, for example, a temperature-responsive culture dish. Examples of usable cell aggregates further include those that do not contain a scaffold material such as a collagen sheet. The cells may be in the form of a cell cluster.

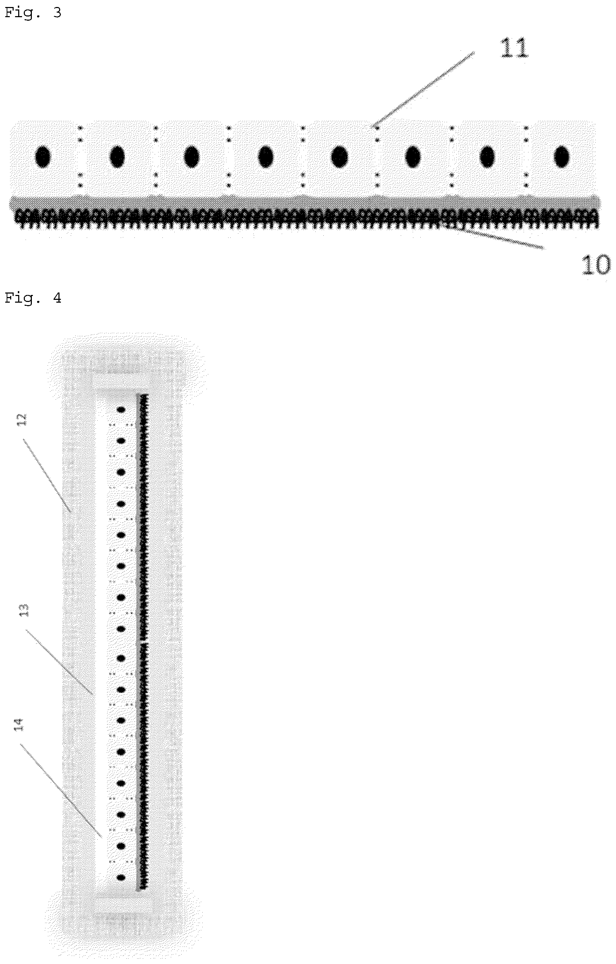

In a preferred embodiment, the cell aggregate is a cell sheet. As used herein, the term "cell sheet" refers to a cell aggregate in which cells are connected to each other by intercellular binding and in the form of a sheet comprising a single layer or a multilayer (preferably a single layer)). The cell sheet is composed of cells and an extracellular matrix, and maintains adhesion between the cells. In a preferred embodiment, as shown in Fig. 3, the cell sheet has a structure in which the sheet-like cells (11) are laminated on a sheet-like extracellular matrix (10). In this embodiment, the cells are preferably disposed as a single layer on the extracellular matrix.

The cell sheet can be obtained, for example, by culturing cells on a stimulus-responsive culture substrate coated with a polymer whose molecular structure changes in response to a stimulus, such as temperature, pH, or light, and changing the surface of the stimulus-responsive culture substrate by changing conditions of the stimulus such as temperature, pH, or light. An example of the stimulus-responsive culture substrate is UpCell (registered trademark), which is a commercially available temperature-responsive culture dish produced by CellSeed Inc. In a preferred embodiment, an extracellular matrix is formed during the cell culture step, and the cells are maintained in an adhered state.

A single cell sheet may be used as the cell aggregate, or two or three cell sheets may be laminated and used in a laminated state as a cell aggregate.

When cell sheets are used as the cell aggregate, the cell sheets may be laminated on a scaffold material such as a collagen sponge to increase ease of operation and used in that state; however, it is not always necessary to use a scaffold material. When cell sheets are used as the cell aggregate, no scaffold material is required to hold the cells, and the immunoisolation device can be produced without intentionally adding a scaffold material. Accordingly, in one embodiment, the present invention provides an immunoisolation device as described above, wherein the cell aggregate is a cell sheet comprising cells and an extracellular matrix, without any intentionally added scaffold material.

The thickness of the cell aggregate is not particularly limited, but is preferably 10 µm or more, and more preferably 20 µm or more. Further, the thickness of the cell aggregate is preferably 300 µm or less, more preferably 290 µm or less, and even more preferably 150 µm or less. A thickness within the above range is preferable because the supply of oxygen and the like to cells is less likely to be hindered.

The animal species from which the cells for use in the cell aggregates are derived may include, for example, mammals such as humans, rats, mice, guinea pigs, marmosets, rabbits, dogs, cats, sheep, pigs, goats, monkeys, chimpanzees, and immunodeficient animals thereof, birds, reptiles, amphibians, fish, insects, etc. When the immunoisolation device of the present invention is used for the treatment of a human, cells of human origin are preferably used. When the immunoisolation device is used for human treatment, the cells may be taken from the patient themselves or from other persons, or from a commercially available cell line.

Examples of cells used in cell aggregates include somatic cells that constitute a living organism (cardiomyocytes, hepatic parenchymal cells, kidney cells, adrenal cortical cells, epidermal cells, vascular endothelial cells, mucosal cells, pancreatic islet cells, etc.), germ cells (sperm, eggs, etc.), stem cells (mesenchymal stem cells, ES cells, iPS cells, etc.), precursor cells, cells isolated from living organisms and having acquired immortalizing ability and maintained stably in vitro, cells isolated from living organisms and artificially genetically modified, and cells isolated from living organisms and having artificially replaced nuclei.

In a preferred embodiment, the cells for use in the cell aggregate include those that release a biologically active substance from the immunoisolation device. Examples include mesenchymal stem cells, pancreatic islet cells, pancreatic islet-like insulin-producing cells, pituitary hormone-producing cells, and lysosomal enzyme-producing cells.

The cell aggregate may be composed of multiple kinds of cells mentioned above.

### Immune Isolation Layer

The immunoisolation device of the present invention comprises an immunoisolation layer that covers the cell aggregate. In a typical embodiment, from the viewpoint of obtaining a sufficient immunoisolation effect, it is preferable that the immunoisolation layer in the device of the present invention covers the entire surface of the cell aggregate. For the same reason, the immunoisolation layer preferably does not have any pores such as pinholes penetrating the membrane.

The immunoisolation layer preferably comprises a porous membrane or a fiber structure. More preferably, the immunoisolation layer is preferably an immunoisolation multilayer comprising a porous membrane or fiber structure and a hydrogel. The immunoisolation layer may be an immunoisolation multilayer comprising a porous membrane and a fiber structure, or may be an immunoisolation multilayer comprising a porous membrane, a fiber structure, and a hydrogel.

### Porous Membrane

The porous membrane of the immunoisolation layer is a membrane having multiple pores. Being a porous membrane can be confirmed by scanning electron microscope (SEM) images or transmission electron microscope (TEM) images of the cross-section of the membrane. The porous membrane is preferably a semi-permeable membrane.

The thickness of the porous membrane is not particularly limited, but is preferably 300 µm or less, more preferably 15 µm to 290 µm, and even more preferably 30 µm to 150 µm. A thickness within the above range is preferable because the strength of the immunoisolation layer is maintained while the supply of oxygen and other substances to the cells is less likely to be hindered.

The average pore size of the porous membrane is not particularly limited, but is preferably 0.01 µm to 10 µm, more preferably 0.01 µm to 5 µm, and even more preferably 0.01 to 3 µm. The average pore size can be determined from SEM images or TEM images. For example, the surface of the porous membrane is observed using SEM, and 50 pores are randomly selected from pores formed on the surface. The major diameter of each pore is measured, and the average of the major diameters of 50 pores is calculated to obtain the average pore size.

The maximum pore size of the porous membrane is not particularly limited, but is preferably 0.01 µm to 10 µm, more preferably 0.01 µm to 5 µm, and even more preferably 0.01 µm to 4 µm. When the maximum pore size is within the above range, the invasion of immune response cells into the device can be inhibited, while nutrients, such as amino acids, vitamins, inorganic salts, glucose, and like carbon sources, and physiologically active substances such as oxygen, carbon dioxide, cytokines, hormones, and insulin, are allowed to permeate sufficiently. The maximum pore size can be determined from SEM images or TEM images. For example, the surface of the porous membrane is observed using SEM, and 50 pores are randomly selected from pores formed on the surface. The major diameter of each pore is measured, and the maximum value among the 50 major diameters is defined as the maximum pore size.

From the viewpoint of the necessity of having the functions of inhibiting infiltration of cells from the recipient and also preventing leakage of cells, which are a material to be transplanted, the average pore size or the maximum pore size of the porous membrane is preferably 5 µm or less so as to be smaller than the cell size.

The porous membrane comprises a polymer. Preferably, the porous membrane is substantially composed of a polymer. The polymer may be, for example, a thermoplastic or thermosetting polymer. The polymer may be biocompatible. Specific examples of the polymer include ethylene-vinyl alcohol copolymers, polysulfones, cellulose acetates, nitrocellulose, sulfonated polysulfones, polyethersulfones, polyacrylonitrile, styreneacrylonitrile copolymers, styrene-butadiene copolymers, polyvinyl alcohols, polycarbonates, organosiloxane-polycarbonate copolymers, polyester carbonates, organopolysiloxanes, polyphenylene oxides, polyamides, polyimides, polyamideimides, polybenzimidazoles, and polytetrafluoroethylene (PTFE). In view of solubility, optical properties, electrical properties, strength, elasticity, etc., the polymer may be a homopolymer or may be a copolymer, a polymer blend, a polymer alloy, or the like. The polymer that constitutes the porous membrane may contain a hydrophilic polymer such as polyvinylpyrrolidone, hydroxypropyl cellulose, hydroxyethyl cellulose, or polyethylene glycol. Combining hydrophilic and hydrophobic polymers can improve biocompatibility.

The ethylene-vinyl alcohol copolymer can usually be obtained by saponifying an ethylene-vinyl ester copolymer. The production and saponification of the ethylene-vinyl ester copolymer can be performed by known methods. A representative example of the vinyl ester is vinyl acetate, but other fatty acid vinyl esters such as vinyl formate, vinyl propionate, vinyl valerate, vinyl caprate, vinyl laurate, vinyl stearate, vinyl pivalate, and vinyl versatate are also usable.

The ethylene unit content of the ethylene-vinyl alcohol copolymer is preferably 20 mol% or more, and more preferably 25 mol% or more. Further, the ethylene unit content of the ethylene-vinyl alcohol copolymer is preferably 60 mol% or less, more preferably 55 mol% or less, and even more preferably 50 mol% or less.

The degree of saponification of the ethylene-vinyl alcohol copolymer is preferably 80 mol% or more, more preferably 90 mol% or more, and even more preferably 95 mol% or more. Further, the degree of saponification of the ethylene-vinyl alcohol copolymer may be 100 mol% or less, or may be 99.99 mol% or less. The degree of saponification of the ethylene-vinyl alcohol copolymer can be calculated by performing ¹H-NMR measurement and measuring the peak area of hydrogen atoms contained in the vinyl ester structure and the peak area of hydrogen atoms contained in the vinyl alcohol structure.

As long as the object of the present invention is not impaired, the ethylene-vinyl alcohol copolymer may further contain units derived from monomers other than ethylene, vinyl esters, and saponified products thereof. When the ethylene-vinyl alcohol copolymer has such other monomer units, the content of such other monomer units based on the total monomer units of the ethylene-vinyl alcohol copolymer is preferably 30 mol% or less, more preferably 20 mol% or less, even more preferably 10 mol% or less, and particularly preferably 5 mol% or less. When the ethylene-vinyl alcohol copolymer contains units derived from such other monomers, the lower limit of the content may be 0.05 mol% or 0.10 mol%. Examples of other monomers include alkenes such as propylene, butylene, pentene, and hexene; ester group-containing alkenes or saponified products thereof, such as 3-acyloxy-1-propene, 3-acyloxy-1-butene, 4-acyloxy-1-butene, 3,4-diacyloxy-1-butene, 3-acyloxy-4-methyl-1-butene, 4-acyloxy-2-methyl-1-butene, 4-acyloxy-3-methyl-1-butene, 3,4-diacyloxy-2-methyl-1-butene, 4-acyloxy-1-pentene, 5-acyloxy-1-pentene, 4,5-diacyloxy-1-pentene, 4-acyloxy-1-hexene, 5-acyloxy-1-hexene, 6-acyloxy-1-hexene, 5,6-diacyloxy-1-hexene, and 1,3-diacetoxy-2-methyleneprylene; unsaturated acids such as acrylic acid, methacrylic acid, crotonic acid, and itaconic acid, or anhydrides, salts, or mono- or di-alkyl esters thereof; nitriles such as acrylonitrile and methacrylonitrile; amides such as acrylamide and methacrylamide; olefin sulfonic acids such as vinyl sulfonic acid, allyl sulfonic acid, methallylsulfonic acid, or salts thereof; vinyl silane compounds such as vinyltrimethoxysilane, vinyltriethoxysilane, vinyltri(β-methoxy-ethoxy)silane, and γ-methacryloxypropylmethoxysilane; alkyl vinyl ethers, vinyl ketones, N-vinylpyrrolidone, vinyl chloride, vinylidene chloride, and the like.

The ethylene-vinyl alcohol copolymer may be post-modified by urethanization, acetalization, cyanoethylation, oxyalkylenation, or the like.

Such ethylene-vinyl alcohol copolymers may be used alone or in a combination of two or more.

The polymer for forming the porous membrane is preferably a highly biocompatible material that is unlikely to cause adhesion to the recipient's tissue around the transplantation site or incur inflammation, etc. The porous membrane preferably contains at least one member selected from the group consisting of ethylene-vinyl alcohol copolymers and cellulose acetate.

The porous membrane may be composed of one kind of porous membrane or may be composed of two or more kinds of porous membranes laminated on each other. When the porous membrane comprises two or more porous membranes, the porous membranes may be directly laminated, or a hydrogel or a fiber structure may be interposed between the two porous membranes.

In a preferred embodiment, the porous membrane is a single layer formed of one composition. In one preferred embodiment, the porous membrane does not have a multilayer lamination structure.

### Fiber Structure

The fiber structure of the immunoisolation layer is, for example, a nonwoven fabric, a woven fabric, or a knitted fabric. The fiber structure is preferably a nonwoven fabric. The fiber structure is formed by bonding or entangling fibers together by heat, mechanical, or chemical action. The basis weight (weight per unit area) can be adjusted by adjusting the fiber diameter and/or amount of fibers, so that in addition to the strength, the permeability and/or filtration can be controlled. The fiber structure preferably has a basis weight of 10 to 100 g/m². Considering the diffusion efficiency of physiologically active substances released from the material to be transplanted, the thickness of the fiber structure is preferably 300 µm or less, and is more preferably is 200 µm or less and as thin as possible.

Examples of fiber materials of the fiber structure include gelatin, collagen, chitin, chitosan, fibronectin, dextran, cellulose, polyethylene (PE), polypropylene (PP), polyurethane, polyamide, polyester, polyvinyl alcohol (PVA), ethylene-vinyl alcohol copolymers, polylactic acid, polyglycolic acid, polylactic acid-polyglycolic acid copolymers, PVAs modified with a monomer such as methacrylic-modified PVA and acrylic-modified PVA, polycaprolactone, polyglycerol sebacic acid, polyhydroxyalkanoic acid, polybutylene succinate, polymethylene carbonate, cellulose diacetate, cellulose triacetate, methylcellulose, propylcellulose, benzyl cellulose, cellulose acetates such as carboxymethyl cellulose, fibroin, and silk. The fiber material of the fiber structure as well as the porous membrane is preferably biocompatible. The fiber structure preferably contains at least one member selected from the group consisting of ethylene-vinyl alcohol copolymers and cellulose acetate. The surface of the fiber structure is preferably smoothed by heat, mechanical, or chemical treatment. When an ethylene-vinyl alcohol copolymer is used, ethylene-vinyl alcohol copolymers described in the explanation of the porous membrane can be preferably used.

### Hydrogel

Examples of hydrosols for producing the hydrogel of the immunoisolation layer include sols that gel in the presence of a metal ion to form hydrogels; sols that gel in response to temperature to form hydrogels; sols that gel in response to pH to form hydrogels; and sols that gel in response to light to form hydrogels. Metal ions and pH are examples of chemical action. In order to gel such hydrosols, an operation such as bringing a metal ion into contact with a hydrosol, adjusting the temperature to gelling conditions, adjusting the pH to gelling conditions, applying light under gelling conditions, or providing a magnetic field under gelling conditions may be performed depending on the characteristics of the gel used.

Examples of hydrogels obtainable by gelling in the presence of a metal ion include alginate gels obtainable by gelling in the presence of a divalent or trivalent metal ion, preferably an alkaline earth metal ion, such as a calcium ion or a magnesium ion; carrageenan gels obtainable by gelling in the presence of a calcium ion and/or a potassium ion; and acrylic acid-based synthesis gels obtainable by gelling in the presence of a sodium ion.

Examples of temperature-responsive hydrogels include a temperature-responsive hydrogel obtainable by crosslinking poly(N-isopropylacrylamide) with polyethylene glycol (trade name: Mebiol Gel), methylcellulose, hydroxypropyl cellulose, a copolymer of lactic acid and ethylene glycol, a triblock copolymer of polyethylene glycol and polypropylene oxide (trade name: Pluronic (registered trademark), poloxamer), agarose, polyvinyl alcohol, and the like.

Examples of pH-responsive hydrogels include alginate gels, chitosan gels, carboxymethyl cellulose gels, and acrylic acid-based synthetic gels.

Examples of photoresponsive hydrogels include a synthesis gel in which azobenzene and cyclodextrin are combined in the skeleton, a gel composed of a supramolecule having fumaric acid amide as a spacer, a gel obtainable by crosslinking or bonding via a nitrobenzyl group, and a gel composed of modified polyvinyl alcohol.

The modified polyvinyl alcohol may be, for example, a (meth)acryloyl group-modified polyvinyl alcohol. The (meth)acryloyl group can be introduced by subjecting a hydroxyl group on the side chain of polyvinyl alcohol to an esterification reaction or an ester exchange reaction with an ethylenically unsaturated group-containing compound in the presence of a base. Examples of the ethylenically unsaturated group-containing compound include (meth)acrylic acid and derivatives thereof, such as (meth)acrylic acid, (meth)acrylic anhydride, (meth)acrylic acid halide, and (meth)acrylic acid ester.

Preferred examples of the hydrogel include polyvinyl alcohol, polyethylene glycol, chitosan, alginate, and the like. The hydrogel preferably contains at least one member selected from the group consisting of polyvinyl alcohols and polyethylene glycols, and more preferably contains a polyvinyl alcohol. The polyvinyl alcohol-based polymer can be produced, for example, by saponification of a polyvinyl ester obtainable by polymerizing a vinyl ester-based monomer, and converting the ester group in the polyvinyl ester to a hydroxyl group.

Examples of the vinyl ester-based monomer include aliphatic vinyl esters such as vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, and vinyl oleate; and aromatic vinyl ester such as vinyl benzoate. These vinyl ester-based monomers may be used alone or in a combination of two or more.

Among the vinyl ester-based monomers, aliphatic vinyl esters are preferred, and vinyl acetate is more preferred in view of production cost. In other words, the polyvinyl ester is preferably polyvinyl acetate obtainable by polymerization of vinyl acetate.

The polyvinyl ester may optionally contain structural units derived from monomers other than vinyl ester monomers as long as the effect of the present invention is not impaired. Examples of such other monomers include α-olefins such as ethylene, propylene, n-butene, and isobutylene; acrylic acid or salts thereof; alkyl acrylates such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives such as acrylamide, N-methylacrylamide, N-ethylacrylamide, N,N-dimethylacrylamide, diacetoneacrylamide, acrylamidopropane sulfonic acid or salts thereof, acrylamidopropyl dimethylamine or salts or quaternary salts thereof, and N-methylolacrylamide or derivatives thereof; methacrylamide derivatives such as methacrylamide, N-methylmethacrylamide, N-ethylmethacrylamide, methacrylamidopropanesulfonic acid or salts thereof, methacrylamidopropyl dimethylamine or salts or quaternary salts thereof, N-methylolmethacrylamide or derivatives thereof; N-vinylamide derivatives such as N-vinylformamide and N-vinylacetamide; vinyl ethers such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles such as acrylonitrile and methacrylonitrile; vinyl halides such as vinyl chloride and vinyl fluoride; vinylidene halides such as vinylidene chloride and vinylidene fluoride; allyl compounds such as allyl acetate and allyl chloride; maleic acid or salts, esters, or acid anhydrides thereof; vinyl silyl compounds such as vinyl trimethoxysilane; and isopropenyl acetate. These can be used alone or in a combination of two or more.

The average polymerization degree of the polyvinyl alcohol is preferably 300 to 10000, more preferably 500 to 5000, even more preferably 1000 to 5000, and particularly preferably 2000 to 5000. A thickness within the above range is preferred in view of permeability of substances and ease of handling in a multilayer formation.

The average polymerization degree of the polyvinyl alcohol in the present specification refers to the average polymerization degree measured according to JIS K 6726:1994. Specifically, the average polymerization degree can be determined from the intrinsic viscosity that is measured in water at 30°C after the raw material PVA has been saponified and purified.

From the viewpoint of improving the water solubility of the polyvinyl alcohol, the degree of saponification of the polyvinyl alcohol is preferably 50 mol% or more, more preferably 60 mol% or more, and even more preferably 65 mol% or more.

From the viewpoint of inhibiting the hydrosol solution from having high viscosity and improving the storage stability of the hydrosol solution, the degree of saponification of the polyvinyl alcohol is preferably 99 mol% or less.

In the present specification, the saponification degree of polyvinyl alcohol means a ratio (mol%) of the number of moles of the vinyl alcohol unit to the total number of moles of the structural unit (e.g., vinyl acetate unit) that can be converted to a vinyl alcohol unit by saponification in the raw material PVA and the vinyl alcohol unit. The degree of saponification can be measured according to JIS K 6726:1994.

The thickness of the hydrogel is not particularly limited, but is preferably 1 to 300 µm, more preferably 5 to 200 µm, and even more preferably 10 to 100 µm.

The hydrogel may be composed of one hydrogel or a laminate of two or more hydrogels. When the immunoisolation device of the present invention comprises two or more hydrogels, the hydrogels may be directly laminated, or a porous membrane or a fiber structure may be interposed between the two hydrogels.

The crosslinking of the hydrogel makes it possible to adjust the permeability, strength, etc. of nutrients such as glucose, physiologically active substances such as insulin, and immune system humoral factors.

The gel strength of the hydrogel is preferably 20 to 300 kPa, and more preferably 50 to 200 kPa. The gel strength can be measured by the following procedure using a tensile tester.

First, the hydrosol solution is poured between two glass plates that are separated from each other by inserting 1 mm-thick spacers therebetween and treated under the predetermined gelling conditions to obtain a gel sheet with a thickness of 1 mm. A test piece is cut out from the gel sheet using a dumbbell cutter specified in JIS K 6251-3. The test piece is set on a tensile tester (model 5566) produced by Instron Co., Ltd., and the breaking stress and breaking strain are measured while acquiring image data. The stress at which the test piece breaks is defined as the gel strength.

### Configuration etc. of Immunoisolation Layer

The immunoisolation layer of the present invention comprises a porous membrane or a fiber structure, wherein the porous membrane or the fiber structure preferably comprises at least one member selected from the group consisting of ethylene-vinyl alcohol copolymers and cellulose acetate. Further, the immunoisolation layer of the present invention is preferably an immunoisolation multilayer comprising a porous membrane or fiber structure and a hydrogel.

Forming a multilayer with a hydrogel increases the strength of the immunoisolation layer as well as improves immunoisolation. Achieving both permeability and immunoisolation can be adjusted by adjusting the pore size of the porous membrane and the gel strength and/or degree of crosslinking of the hydrogel.

Cell infiltration and cell leakage can be inhibited by the porous membrane. However, it is not easy to suppress the infiltration of immune system humoral factors, such as IgG antibodies, while the permeability of necessary physiologically active substances is not reduced. Accordingly, by adjusting the gel strength or crosslinking density of the hydrogel that forms a multilayer with the porous membrane, the infiltration of immune system humoral factors such as IgG antibodies can be inhibited without reducing the permeability of physiologically active substances.

When the fiber structure layer used is a single layer, it is not easy to inhibit the infiltration of immune system humoral factors, such as IgG antibodies, as well as cell infiltration and cell leakage, while the permeability of necessary physiologically active substances is not reduced. Accordingly, by adjusting the gel strength or crosslinking density of the hydrogel that forms a multilayer with the porous membrane, the infiltration of immune system humoral factors such as IgG antibodies, in addition to cell infiltration and cell leakage, can be inhibited without reducing the permeability of physiologically active substances.

The porous membrane (b1), the fiber structure (b2), and the hydrogel (b3) each form a layer, and the layers may be clearly separated at their boundaries, or the boundary between two adjacent layers may not be clearly defined, or two or three different kinds of layers may be integrated to form one layer. For example, when the immunoisolation layer comprises two kinds of layers that are a fiber structure and a hydrogel, the immunoisolation layer may be an immunoisolation multilayer in which the fiber structure and the hydrogel are clearly separated, or a mixed layer of the fiber structure and the hydrogel may be present between the fiber structure and the hydrogel, or the fiber structure and the hydrogel may be completely integrated into one layer. For example, when the immunoisolation layer comprises two kinds of layers that are a fiber structure and a porous membrane, the immunoisolation layer may be an immunoisolation multilayer in which the fiber structure and the porous membrane are clearly separated, or a mixed layer of the fiber structure and the porous membrane may be present between the fiber structure and the hydrogel, or the fiber structure and the porous membrane may be completely integrated into one layer. For example, when the immunoisolation layer comprises two kinds of layers that are a hydrogel and a porous membrane, the immunoisolation layer may be an immunoisolation multilayer in which the hydrogel and the porous membrane are clearly separated, or a mixed layer of the hydrogel and the porous membrane may be present between the hydrogel and the porous membrane, or the hydrogel and the porous membrane may be completely integrated into one layer.

The immunoisolation device according to a particularly preferred embodiment of the present invention comprises any one of the following three multilayer structures (i) to (iii):
(i) A porous membrane is used as a substrate, and a hydrogel is applied to the porous membrane or the porous membrane is impregnated with a hydrogel to form a multilayer structure.
(ii) A fiber structure is used as a substrate, and a hydrogel is applied to the fiber structure or the fiber structure is impregnated with a hydrogel to form a multilayer structure.
(iii) A fiber structure is used as a substrate, and a porous membrane is formed thereon. A hydrogel is further applied to the porous membrane or the porous membrane is impregnated with a hydrogel to form a multilayer structure.

When the immunoisolation membrane (B) comprises multiple layers, the layers may be bonded by an adhesive, heat, pressure, or the like. When adjacent layers are formed of highly compatible materials, the layers can be bonded by sequentially forming the layers.

Specifically, a porous membrane is used as a substrate, and a hydrosol solution is directly applied to the porous membrane and formed into a hydrogel by heat, temperature, light, or chemical action to form a multilayer. Alternatively, using a fiber structure as a substrate, a hydrosol solution is directly applied to the fiber structure and formed into a hydrogel by heat, temperature, light, or chemical action to form a multilayer structure. When a fiber structure such as a nonwoven fabric coated with a hydrogel is used instead of the porous membrane, an immunoisolation layer that has higher material permeability and higher strength than the porous membrane can be formed while the hydrogel ensures immunoisolation.

The hydrosol solution preferably has a solids concentration of 3 to 15 mass%, more preferably 3 to 10 mass%, even more preferably 3 to 8 mass%, and particularly preferably 3 to 5 mass%. A solids concentration within the above range is preferable because the permeability of immune system liquid factors such as IgG can be inhibited while the permeability of substances such as glucose and insulin is maintained.

Alternatively, a hydrogel is used as a substrate, and a polymer solution, which is a raw material for a porous membrane is directly applied to the substrate. The raw material for a porous membrane is solidified by phase separation, which is a phase transition phenomenon, to thereby form a porous membrane. Some raw materials for the porous membrane may require pretreatment to reduce the water content by pre-drying the hydrogel.

In one preferred embodiment of the present invention, a fiber structure with excellent durability is used as a substrate; and either a porous polymer membrane or a hydrogel, or both, are formed on the fiber structure to thereby achieve both a reduction in thickness of the device, which improves the diffusion efficiency of physiologically active substances, and durability due to increased strength, while maintaining the immunoisolation effect.

The thickness of the immunoisolation layer is not particularly limited, but is preferably 10 µm or more and 500 µm or less, more preferably 300 µm or less, even more preferably 200 µm or less, still even more preferably 170 µm or less, and particularly preferably 150 µm or less. Even when the immunoisolation layer is an immunoisolatoin multilayer, the preferable thickness of the immunoisolation layer is also within the above range. Considering the mass diffusion efficiency of the physiologically active substance released from the material to be transplanted, the film thickness of the multilayer membrane is preferably 100 µm or less and as thin as possible.

The outermost layer of the immunoisolation layer is preferably biocompatible in order to prevent the immunoisolation layer from being recognized as foreign matter. The immunoisolation layer is required to have permeability to allow for sufficient permeation of oxygen and nutrients into the material to be transplanted that is present inside of the device. Since it is necessary to block the invasion of immunocompetent cells into the device, a region having a pore size that blocks the permeation of immunocompetent cells is preferably present on the outer surface, inner surface, or inside of the immunoisolation layer throughout the entire immunoisolation layer.

The outermost layer of the immunoisolation layer may be any of a porous membrane, a fiber structure, or a hydrogel, or a mixture of two or three of these. The innermost layer of the immunoisolation layer may be any of a porous membrane, a fiber structure, or a hydrogel, or a mixture of two or three of these. In the present specification, the outermost layer of the immunoisolation layer means the layer that constitutes the outer part of the immunoisolation device of the present invention in the immunoisolation layer, i.e., the layer that contacts with the tissue surrounding the transplantation site (host), and the innermost layer of the immunoisolation layer means the layer that constitutes the part of the immunoisolation layer that contacts the cell aggregate in the immunoisolation device of the present invention (the inner portion).

When the outermost layer of the immunoisolation layer is a porous membrane, the porous membrane is desirably made of a material that is more biocompatible than hydrogel, and also serves to prevent adhesion of the hydrogel to the recipient's tissue at the transplantation site and the induction of inflammation.

When the outermost layer of the immunoisolation layer is a fiber structure, the fiber structure is desirably a material with higher biocompatibility than the hydrogel. Since the outer layer also serves to protect the hydrogel from adhering to the recipient's tissue at the transplantation site and inducing inflammation, etc., the surface of the fiber structure as the outermost layer is desirably smoothed by heat, mechanical, or chemical treatment.

By modifying the hydrogel to inhibit the induction of inflammatory reactions, it is also possible to use the hydrogel as the outermost layer and use the porous membrane as the innermost layer. In that case, it is also possible to load a physiologically active substance on the hydrogel and impart functionality such as induction of angiogenesis.

In one embodiment, the innermost layer of the immunoisolation layer is preferably composed of a hydrogel or a porous membrane on which a cell-adhesive protein and/or a cell-adhesive peptide is immobilized. This configuration allows the cell aggregate to adhere to the innermost layer of the immunoisolation layer, which eliminates the need for a scaffolding material for cell aggregates, thus reducing the overall thickness of the immunoisolation device.

When the inner layer is composed of multiple layers, the layers can be made of the same material or different materials. In the present invention, all of the layers (a single layer or multiple layers) that include the innermost layer but do not include the outermost layer are collectively referred to as the inner layer. Accordingly, in the present invention, both a single innermost layer and layers consisting of multiple layers including the innermost layer can be included in the concept of the "inner layer." For example, when the multilayer has a four-layer structure, the innermost layer of the multilayer can be interpreted as the "inner layer," or the two layers that are the innermost layer and the secondary inner layer can be interpreted as the "inner layer," or the three consecutive layers including the innermost layer can be interpreted as the "inner layer." The inner layer may be any one of a porous membrane, a fiber structure, and a hydrogel, or may be a mixture of two or three of these.

The cell adhesive protein may be, for example, one or more proteins such as gelatin, fibrin, fibronectin, laminin, collagen, retronectin, vitronectin, and elastin. The cell adhesive peptide may be, for example, one or more peptides such as RGD peptide, RGDS peptide, GRGD peptide, and GRGDS peptide.

The method for immobilizing the cell adhesive protein and/or the cell adhesive peptide on the hydrogel or porous membrane is not limited. The immobilization can be achieved, for example, by a known usual method, such as physical adsorption by application of an aqueous cell adhesive protein solution or an aqueous cell adhesive peptide solution. Alternatively, the immobilization can also be achieved by active esterification of a functional group on the hydrogel surface using a condensing agent such as a water-soluble carbodiimide, and then covalently bonding the esterified functional group to an amino group of the cell adhesive protein and/or cell adhesive peptide.

The immobilization amount of the cell adhesive protein and/or cell adhesive peptide is not particularly limited, but is preferably 0.05 µg/cm² or more, and more preferably 0.1 µg/cm² or more. The immobilization amount of the cell adhesive protein and/or the cell adhesive peptide can be measured by immersing a part of the hydrogel or porous membrane in excess PBS (an aqueous solution of PBS tablet (produced by Takara Bio Inc.) in the predetermined amount of ion-exchanged water) overnight and measuring the immobilization amount by the bicinchoninic acid (BCA) method (BCA Protein Assay Kit (produced by Takara Bio Inc.)).

Since the immunoisolation layer of the present invention has sufficient strength and is stably present in the recipient's body and capable of inhibiting the invasion of immunocompetent cells into the device, the invasion of the material to be transplanted that is present inside of the device into the recipient's body can also be inhibited at the same time. Therefore, even if the material to be transplanted is derived from iPS cells, for which canceration is a concern, the material is safe to use.

### Material Permeability of the Immunoisolation Layer

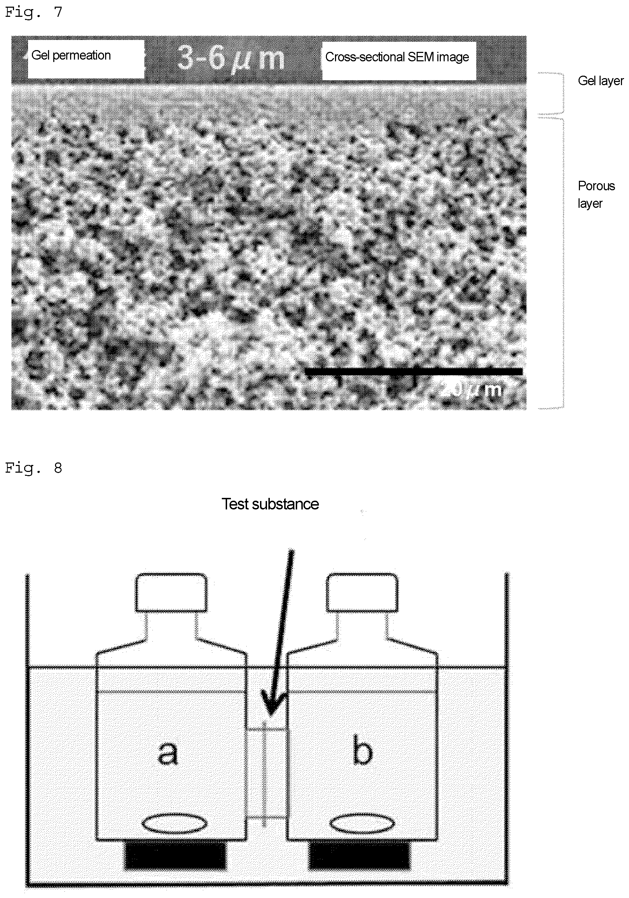

The permeation amount of glucose, insulin, an immune system humoral factor, or the like through the immunoisolation layer can be measured by inserting the immunoisolation layer at the connection site between two glass chambers having the same volume, pouring a sample solution of a known concentration of insulin or the like into chamber a, pouring the same amount of water into chamber b, and quantifying, with stirring at 37°C, the amount of insulin or the like contained in the liquid sampled from chamber b after a certain period of time by ELISA or the like (Fig. 8). Note that the amounts of liquids in chambers a and b are adjusted to be equal when the sample solution is poured into chamber a.

The permeability of glucose, insulin, immune system humoral factors, or the like through the immunoisolation layer is expressed as a percentage that represents what the amount of each substance having permeated into chamber b, as measured by the above method after 20 hours, is relative to the concentration at which the equilibrium is reached, i.e., half the concentration of the substance poured into chamber a. More specifically, for example, the permeability can be obtained according to the following formula: Permeability (%) of each substance = Concentration of each substance in chamber b after 20 hours of measurement / (Concentration of each substance in chamber a at the start of measurement ÷ 2) × 100

The insulin permeability and glucose permeability of the immunoisolation layer of the present invention are preferably 50% or more, more preferably 90% or more, and even more preferably 95% or more.

The immune system humoral factor permeability of the immunoisolation layer of the present invention is preferably 30% or less, and more preferably 10% or less.

The material permeability can be controlled by the pore size of the porous membrane or the gel strength and degree of crosslinking of the hydrogel. In order to exhibit the immune isolation function, it is desirable that the pore size of the porous membrane is equal to or smaller than the size that does not allow permeation of cells. It is desirable that the hydrogel does not inhibit the permeation of physiologically active substances but can inhibit the permeation of immune response factors such as cells and antibodies.

Examples of immunoresponsive cells include macrophages, cytotoxic T cells, natural killer cells, dendritic cells, and helper T cells. Examples of immune system humoral factors include antibodies, complements, and cytokines.

### Immunoisolation Device

The immunoisolation device according to a preferred embodiment of the present invention is in the form of a bag, a tube, a cylinder, a rectangular tube, a sphere, a cube, a rectangular solid, a sheet, or hollow fibers. A cell aggregate is enclosed in the immunoisolation device. In addition to the cell aggregate, physiologically active substances, such as enzymes, hormones, cytokines, and drugs, may also be enclosed.

The immunoisolation device can be produced in the following manner. After an immunoisolation layer is disposed around the cell aggregate to cover the cell aggregate with the immunoisolation layer, the peripheral portion is sealed by heat fusion to form the device into a pouch-like shape. Alternatively, after an opening is provided in the device prepared in advance using an immunoisolation layer and a cell aggregate is inserted from the opening, the opening is closed to thereby block the invasion of immunoresponsive cells and immune system humoral factors from the opening. Since oxygen and nutrients can permeate through the immunoisolation layer in portions other than the opening, the opening can be closed to inhibit the permeation of substances, including nutrients.

Further, alternatively, the immunoisolation device can also be produced by allowing a cell aggregate to adhere to the innermost layer side of the immunoisolation layer and then disposing the immunoisolation layer in such a manner that the cell aggregate is present inside of the device, followed by sealing by heat fusion. In heat fusion, a resin can be interposed between the immunoisolation layers and then heat fusion can be performed. By performing heat fusion in the state that a resin is interposed, heat fusion can be easily performed even when the innermost layers of the upper and lower immunoisolation layers in the immunoisolation device are both a hydrogel (when the hydrogels of the upper and lower immunoisolation layers are facing each other).

After the immunoisolation device of the present invention is implanted in a living body, the material transplanted whose function has been reduced may be removed, and a new functional material to be transplanted may be introduced. This operation can be repeated. The immunoisolation device can be repeatedly used to introduce a material to be transplanted. The immunoisolation device may be removed together with the material transplanted.

The immunoisolation device according to a preferred embodiment of the present invention preferably has shape retention in order to have sufficient strength in a living body.

In order to maintain the functionality as the device, the fiber structure, porous membrane, and hydrogel are preferably materials that are excellent in safety and biocompatibility.

In order to prevent adhesion to the surrounding tissue and prevent fibrosis, the immunoisolation layer is desirably composed of a material with excellent biocompatibility. When the immunoisolation membrane is composed of multiple materials, it is desirable that a material with excellent biocompatibility is disposed at the transplantation side, i.e., on the contact surface of the outermost layer that comes into contact with the transplantation site in a recipient. Examples of the material having excellent biocompatibility are ethylene-vinyl alcohol copolymers. As ethylene-vinyl alcohol copolymers, those described in the explanation of the porous membrane can be preferably used.

Preferred embodiments of the present invention are described below with reference to the drawings.

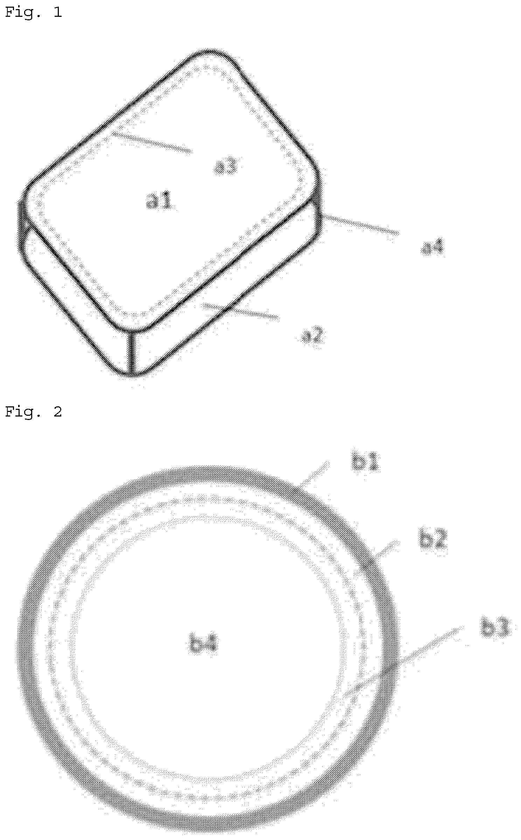

Fig. 1 is a schematic view of a device produced by forming an immunoisolation layer into a bag shape. Fig. 2 is a schematic view of a device produced by forming the immunoisolation layer into a tubular shape. The bag-shaped device (Fig. 1) is formed by applying heat, ultrasound, high frequency, or electron beam to fuse immunoisolation multilayers (a1 and a2) shown below with a certain distance (a4) therebetween (a3) to ensure a space for enclosing a cell aggregate therein. Spacers may be provided to ensure a certain distance (a4).

The tubular device (Fig. 2) is composed of immunoisolation layers (b1, b2, and b3) formed into a tubular shape. A cell aggregate is enclosed inside of the tubular portion (b4) and heat, ultrasound, high frequency, electron beam, or the like is applied to fuse and seal both ends of the tubular layers to form the tubular device.

As shown in Fig. 1 or 2, in the conceptual diagrams, the device is in a bag or tubular shape, and the material to be transplanted, such as cells or a cell cluster, is enclosed as a cell aggregate in the device.

Fig. 3 is a conceptual diagram of a cell sheet. The cell sheet is composed of cells (11), which are a material to be transplanted, and an extracellular matrix (10).

Fig. 4 is a conceptual diagram of an immunoisolation device comprising a cell sheet enclosed therein. It is a conceptual diagram showing that a cell sheet (14) prepared using cells or a cell cluster to be transplanted is inserted into the device. The immunoisolation device is formed into a pouch-like shape by using an immunoisolation multilayer wherein the outermost layer is a porous membrane (12) and the innermost layer is a hydrogel (13). The porous membrane is preferably formed of an ethylene-vinyl alcohol copolymer or the like. The hydrogel of the innermost layer is preferably a polyvinyl alcohol or the like.

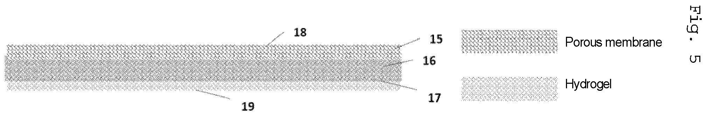

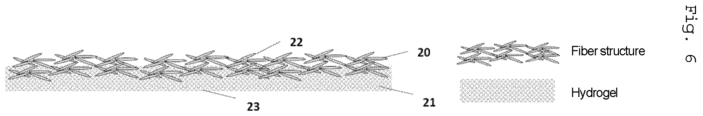

The immunoisolation layers shown in Figs. 5 and 6 are composed of a combination of multiple materials shown below. The outermost layer is in contact with the recipient's tissue at the transplantation site, and the innermost layer is in contact with a cell aggregate. Such immunoisolation layers are formed into a bag shape (Fig. 1) or a tubular shape (Fig. 2), and a cell aggregate having cells or a cell cluster, which is a material to be transplanted, immobilized inside thereof is enclosed therein, thus producing an immunoisolation device.

Fig. 5 shows an immunoisolation multilayer composed of a porous membrane (15) and a hydrogel (17). The hydrogel (17) is applied to the porous membrane (15) or the porous membrane (15) is impregnated with the hydrogel (17) to form a multilayer (16), wherein the outermost layer surface (18) is composed of a porous membrane and the innermost layer surface (19) is composed of a hydrogel.

Fig. 6 shows an immunoisolation multilayer composed of a fiber structure (20) and a hydrogel (21). The hydrogel (21) is applied to the fiber structure (20) or the fiber structure (20) is impregnated with the hydrogel (21) to form a multilayer, wherein the outermost layer surface (22) is composed of the fiber structure, and the innermost layer surface (23) is composed of the hydrogel.

### Examples

The present invention is described in more detail below based on Examples.

### Production of Immunoisolation Multilayer

### Production Example 1

A porous membrane formed using an ethylene-vinyl alcohol copolymer (referred to below as EVOH) and a hydrogel containing a methacryloyl-modified polyvinyl alcohol (referred to below as MA-PVA) as a main component were laminated to form a multilayer by the following procedure.
(1) A porous membrane (average pore size: 1.8 µm, maximum pore size: 3.4 µm, thickness: 100 µm) was produced by subjecting EVOH (EVAL (registered trademark), F101A, produced by Kuraray Co., Ltd.) to a polymer phase separation reaction.
(2) Lithium phenyl(2,4,6-trimethylbenzoyl)phosphinate was added as a water-soluble photoradical polymerization initiator to a 10 mass% aqueous solution of MA-PVA (average degree of polymerization: 1700, saponification degree: 98.0 to 99.0 mol%, methacryloyl modification rate: 1.2 mol%) to achieve a concentration of 0.1 mass%, thus preparing a sol.
(3) This sol was applied to a PET film to a thickness of 50 µm using a bar coater.
(4) The porous membrane was placed on the sol, and the sol and the porous membrane were tightly adhered to each other using a laminator.
(5) The sol surface was then placed on top, and subjected to irradiation with 365 nm light at an intensity of 15 mW/cm² for 3 minutes to form a hydrogel on the porous membrane, thus producing an immunoisolation multilayer. Fig. 7 shows an SEM image of a cross-section of the obtained immunoisolation multilayer. The hydrogel was found to have permeated the porous membrane to a depth of about 3 to 6 µm.

### Production Example 2

Using a 4 mass% aqueous solution of MA-PVA (average degree of polymerization: 3500, saponification degree: 87.0 to 89.0 mol%, methacryloyl modification rate: 1.2 mol%), a hydrogel was formed on a porous membrane in the same manner as in Production Example 1 to prepare an immunoisolation multilayer.

### Material Permeability Test

The glucose, insulin, and IgG permeabilities of the porous membrane produced in Production Example 1 and the immunoisolation multilayers obtained in Production Example 1 or 2 were measured in the following manner.
(1) The porous membrane or the immunoisolation multilayers were each placed between chambers a and b (Fig. 8). An aqueous solution (65 mL) containing insulin (30 U/L), glucose (5 mg/mL), and IgG (0.5 µg/mL) was prepared in chamber a, whereas water (65 mL) was placed in chamber b.
(2) Stirring was performed with a stirrer at a constant temperature of 37°C 20 hours later, liquid was sampled from chamber b, and the concentration changes of insulin, glucose, and IgG were measured by ELISA.
(3) The permeability of each substance was calculated from the results of (2) (Table 1).
Permeability (%) of each substance = Concentration of each substance in chamber b after 20 hours of measurement / (Concentration of each substance in chamber a at the start of measurement ÷ 2) × 100

**Table 1**

| | | Permeability after 20 hours (%) | | |
|---|---|---|---|---|
| | | Glucose | Insulin | IgG |
| Reference Example 1 | EVOH porous membrane | 72 | 25 | 3 |
| Production Example 2 | Immunoisolation multilayer | 60 | 8 | 0 |
| Production Example 3 | Immunoisolation multilayer | 69 | 17 | 0 |

### Immobilization of Cell Adhesive Protein onto Hydrogel Production Example 3

Collagen, which is a cell-adhesive protein, was immobilized (covalently bonded) on the hydrogel surface of an immunoisolation multilayer by the following procedure.
(1) Twenty sheets of an immunoisolation multilayer (size: 24 mm × 32 mm) having an MA-PVA hydrogel formed on one side of an EVOH porous membrane were prepared by the method described in Production Example 2.
(2) 20 mL of MES (2-morpholinoethanesulfonic acid) buffer was placed in a Petri dish, and the immunoisolation multilayer prepared in (1) was immersed in the buffer and shaken at 100 rpm for 30 minutes.
(3) 10 mL of MES buffer was placed in a beaker separately prepared, and 100 mg of N-hydroxysuccinimide (produced by Fujifilm Wako Pure Chemical Corporation, referred to below as NHS) and 170 mg of 1-ethyl-3-(3-dimethylaminopropyl)carbodiimide hydrochloride (produced by Dojindo Laboratories, Ltd., referred to below as WSC) were dissolved in the buffer to prepare an NHS/WSC solution.
(4) After the MES buffer was removed from the Petri dish used in (2), 5 mL of the NHS/WSC solution was added to the Petri dish and shaken at 100 rpm at room temperature for 60 minutes.
(5) The NHS/WSC solution was removed from the Petri dish, and the immunoisolation multilayer was immersed in 20 mL of MES buffer and washed by shaking at room temperature.
(6) Next, a collagen solution (Cellmatrix (registered trademark) Type I-C, Nitta Gelatin Inc.) was diluted with MES buffer to a concentration of 0.2 mg/mL. The immunoisolation multilayer was immersed in 20 mL of this solution and shaken at 100 rpm at room temperature for 20 hours.
(7) The immunoisolation multilayer was then removed, immersed in 20 mL of distilled water, and washed by shaking at 100 rpm at room temperature for 5 minutes.
(8) A release PET film (Cosmopeel (registered trademark) model: E7004, produced by Toyobo Co., Ltd.) was placed on a glass plate. The immunoisolation multilayer was placed on the film, and a release PET film and a glass plate were further placed thereon. The resulting product was dried under reduced pressure at room temperature for 20 hours to obtain an immunoisolation multilayer having collagen immobilized on the surface of the hydrogel.
(9) A part of the hydrogel was immersed overnight in excess PBS (an aqueous solution of a PBS tablet (produced by Takara Bio Inc.) in a predetermined amount of ion-exchanged water). The amount of immobilized collagen was measured by the bicinchoninic acid (BCA) method (BCA Protein Assay Kit (produced by Takara Bio Inc.)) and was found to be 0.5 µg/cm².

### Cell sheet production

### Production Example 4

A co-cultured cell sheet (circular, diameter: 8 mm, thickness: about 100 µm) of a rat pancreatic β cell line capable of secreting insulin-Gaussia luciferase (iGL cells, produced by Cosmo Bio Co., Ltd.) and human adipose-derived mesenchymal stem cells (hASCs, produced by Lonza K.K.) was produced by the following procedure.
(1) 45 × 10⁴ iGL cells and 40 × 10⁴ hASCs were seeded on a temperature-responsive culture dish (12-well multiwell "UpCell" (registered trademark), produced by CellSeed Co., Ltd.) and cultured in a 37°C incubator for 24 hours.
(2) The temperature-responsive culture dish was then removed from the incubator and allowed to stand at room temperature (about 20°C) for 10 minutes to peel off the cells that had adhered and spread onto the bottom surface of the culture dish, thereby obtaining a co-cultured cell sheet.

iGL cells refer to cells whose parent line is rat pancreatic β cell line INS-1E and that have a gene of a fusion protein of insulin and secretory Gaussia luciferase inserted therein. iGL cells react with coelenterazine (CTZ), which is a luminescent substrate, and emit light. Using this luminescence intensity, the insulin releasing ability can be measured.

### Production of Immunoisolation Device: Enclosure of Cell Sheet in Immunoisolation Layer

### Example 1

A collagen sponge (Pelnac (registered trademark), product number: PN-S82060, produced by Gunze Limited) was cut out into a 1 cm square. The cell sheet prepared in Production Example 4 was placed on the center of the cut collagen sponge. This collagen sponge was covered in such a manner that the sponge was interposed between the immunoisolation multilayers prepared in Production Example 2. The peripheral portion of the immunoisolation multilayers was processed into a pouch-like shape by heat sealing, thus producing a strip-shaped cell sheet-enclosed device with a size of 2 cm × 3 cm.

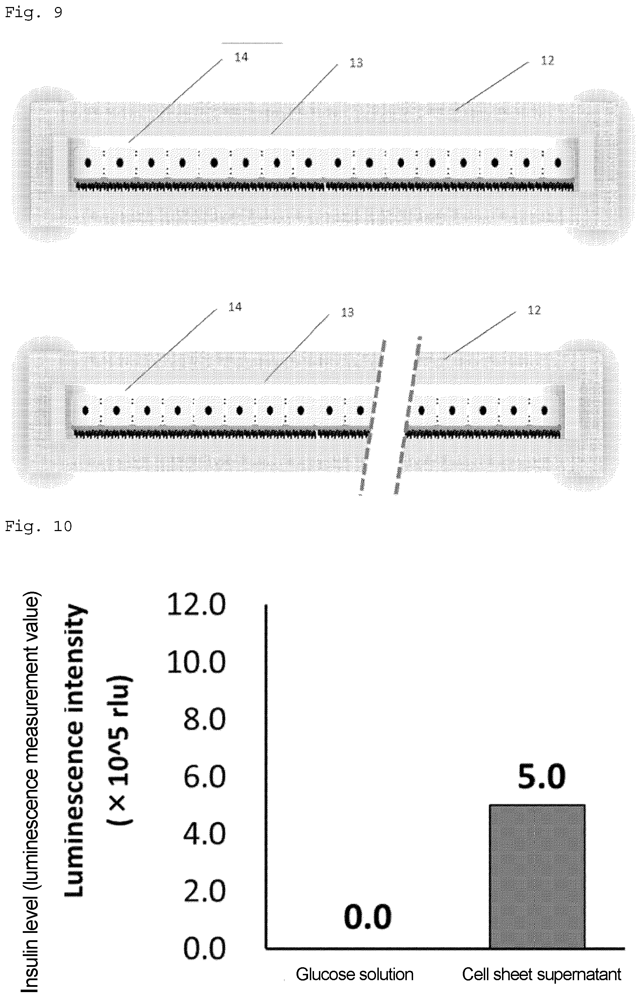

In the above process, the cell sheet was interposed between the immunoisolation multilayers in such a manner that of the two sides of each immunoisolation multilayer, i.e., the MA-PVA hydrogel side and the EVOH porous membrane side, the MA-PVA hydrogel side of each immunoisolation multilayer faced the cell sheet (so that the EVOH porous membrane was exposed to the surface of the device). Fig. 9 shows a conceptual diagram of this device. The dashed line in Fig. 9 indicates that a part of the device is omitted from the drawing.

### Immunoisolation Functionality Test

The cell sheet-enclosed device prepared in Example 1 was transplanted into SD rats (wild rats with immune function), and the immunoisolation performance of the device was confirmed based on cell engraftment and viability.

### 1) Transplantation of Immunoisolation Device

The rat was subjected to abdominal section, and the cell sheet-enclosed device was inserted between the hepatic lobes of the liver and fixed with fibrin glue (product name: Bolheal Tissue Sealant, produced by KM Biologics).

### 2) Functionality Evaluation

### 2-1) Insulin Release Ability in Vitro

On day 10 after the transplantation, the device was removed and then the cell sheet was removed. The cell sheet was immersed in a 20 mM glucose solution for 2 hours, and the supernatant was collected. The insulin release ability of iGL cells was compared based on the fluorescence intensity of the supernatant of the cell sheet.

As shown in Fig. 10, the results confirmed that the cell sheet supernatant had a luminescence intensity of iGL cells significantly correlated with insulin release ability.

### 2-2) Histopathological Evaluation

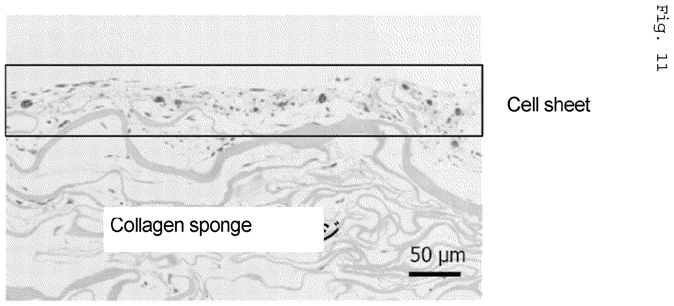

On day 10 after the implantation, tissue sections of each device were prepared and subjected to HE staining and fluorescent immunostaining using insulin antibody. Figs. 11 and 12 show the results of histopathological evaluations.

In the group into which the device produced in Example 1 was transplanted, the survival of insulin-producing cells was confirmed and no inflammatory cell infiltration was confirmed.

The above results confirmed that the device has immunoisolation effects.

### Example 2

The cell sheet prepared in Production Example 4 was placed on the collagen-immobilized hydrogel surface of the immunoisolation multilayer prepared in Production Example 3 and placed in a 60 mm dish. 5 mL of culture medium ("DMEM/F12 GlutaMAX" (registered trademark), produced by Thermo Fisher Scientific, Inc.) was injected into the dish. The 60 mm dish was placed in an incubator at 37°C. After 24 hours, the dish was removed from the incubator and visually observed. The cell sheet was found to have adhered to the surface of the immunoisolation multilayer.

Further, the cells were labeled with a red fluorescent reagent (Cell Explorer (registered trademark), Live Cell Tracking Orange Fluorescence, produced by AAT Bioquest, Inc.) and observed under a stereo fluorescence microscope. The cells around the cell sheet were found to have adhered to the surface of the immunoisolation multilayer and extended. The results clearly show that the use of the collagen-immobilized immunoisolation multilayer allows a cell sheet to directly adhere to the immunoisolation multilayer without using a collagen sponge, thus making the layer of the device thinner.

### Industrial Applicability

The present invention relates to a transplantation device for use in cell transplantation therapy and the like, and in particular to an immunoisolation device for protecting a material to be transplanted from immune rejection reactions.

The immunoisolation device is assumed to be mainly used as a regenerative medicine product for cell transplantation therapy. However, the immunoisolation device is also applicable to the transplantation of physiologically active substances other than cells, such as enzymes, hormones, and drugs.

### Explanation of Reference Symbols

a1 Immunoisolation layer
a2 Immunoisolation layer
a3 Fusing
A4 Certain distance
b1 Immunoisolation layer
b2 Immunoisolation layer
b3 Immunoisolation layer
b4 Tubular interior
10 Extracellular matrix
11 Cells
12 Porous membrane
13 Hydrogel
14 Cell sheet
15 Porous membrane
16 Impregnation and fixation
17 Hydrogel
18 Outermost layer surface
19 Innermost layer surface
20 Fiber structure
21 Hydrogel
22 Outermost layer surface
23 Innermost layer surface

## Claims

1. An immunoisolation device comprising a sheet-like cell aggregate and an immunoisolation layer,
the sheet-like cell aggregate comprising cells and an extracellular matrix, and
the immunoisolation layer covering the cell aggregate.

2. The immunoisolation device according to claim 1, wherein the cell aggregate is a cell sheet comprising the cells and the extracellular matrix.

3. The immunoisolation device according to claim 1 or 2, wherein the cell aggregate has a thickness of 300 µm or less.

4. The immunoisolation device according to any one of claims 1 to 3, wherein
the immunoisolation layer comprises a porous membrane or a fiber structure, and
the porous membrane or the fiber structure comprises at least one member selected from the group consisting of ethylene-vinyl alcohol copolymers and cellulose acetate.

5. The immunoisolation device according to claim 4, wherein the immunoisolation layer is an immunoisolation multilayer comprising the porous membrane or the fiber structure, and a hydrogel.

6. The immunoisolation device according to claim 5, wherein
the outermost layer of the immunoisolation multilayer is the porous membrane or the fiber structure, and
the innermost layer of the immunoisolation multilayer is the hydrogel.

7. The immunoisolation device according to claim 5, wherein
the outermost layer of the immunoisolation multilayer is the hydrogel and
the innermost layer of the immunoisolation multilayer is the porous membrane or the fiber structure.

8. The immunoisolation device according to any one of claims 1 to 7, wherein
the immunoisolation layer comprises multiple layers, and
the innermost layer of the immunoisolation layer is a hydrogel or a porous membrane that has a cell-adhesive protein and/or a cell-adhesive peptide immobilized.

9. The immunoisolation device according to any one of claims 5 to 8, wherein the hydrogel comprises at least one member selected from the group consisting of polyvinyl alcohols and polyethylene glycol.
